Europäisches Patentamt

European Patent Office

Office européen des brevets

(11) Publication number: **0 145 873**

**B1**

(12) EUROPEAN PATENT SPECIFICATION

(45) Date of publication of patent specification: 04.01.89

(21) Application number: **84112091.8**

(22) Date of filing: **09.10.84**

(51) Int. Cl.⁴: **A 61 K 9/00,** A 61 K 31/23,
A 61 K 47/00

(54) Transfusion emulsion.

(30) Priority: 16.12.83 JP 237517/83
17.04.84 JP 77188/84

(43) Date of publication of application:
26.06.85 Bulletin 85/26

(45) Publication of the grant of the patent:
04.01.89 Bulletin 89/01

(84) Designated Contracting States:
DE FR GB SE

(56) References cited:
US-A-3 169 094
US-A-3 198 704

CHEMICAL ABSTRACTS, vol. 73, 1970, page
144, no. 22875y, Columbus, Ohio, US; T.
SASAKI: "Effect of gamma-linolenic
triglyceride on the lipid metabolism in rats on a
fat-free diet" & NAGOYA J. MED. SCI. 1970,
32(2), 215-231

CHEMICAL ABSTRACTS, vol. 87, 1977, page 36,
no. 123139f, Columbus, Ohio, US; S.V.
BASHARIN et al.: "Some properties of
emulsions of linseed oil in water" & UCH. ZAP.,
YAROSL. GOS. PEDAGOG. INST. 1976, 154,
101-106

(73) Proprietor: TERUMO KABUSHIKI KAISHA
trading as TERUMO CORPORATION
44-1, 2-chome, Hatagaya Shibuya-Ku
Tokyo 151 (JP)

(72) Inventor: Wakabayashi, Toshio
30-30-2, 5-chome Nagayama
Tama-shi Tokyo (JP)
Inventor: Ozawa, Shinji Terumo Kabushiki
Kaisha
Honmachiryo 25-2, 5-chome Honmachi
Shibuya-ku Tokyo (JP)
Inventor: Kunimasa, Megumi
19-12, 2-chome Takeoka
Kiyose-shi Tokyo (JP)

(74) Representative: Henkel, Feiler, Hänzel & Partner
Möhlstrasse 37
D-8000 München 80 (DE)

(56) References cited:
Römpp Chemie-Lexikon, p. 5988

CTFA Cosmetic Ingredient Dictionary, p. 298

**Description**

The present invention relates to an improved transfusion emulsion.

More particularly, it is concerned with a transfusion emulsion in which 10—50 w/v% of the fat component is an α-linolenic acid ester.

The transfusion emulsion of the invention is administered to patients, usually by drip infusion into the vein, for nutritional supplement. Moreover, the transfusion emulsion of the invention is useful as an antithrombotic or cholesterol-lowering agent.

The transfusion emulsion is an emulsion preparation composed of fat, emulsifier and water. There is employed in the prior-art transfusion emulsions purified soybean oil or purified safflower oil as the fat component (see for example US—A—3169094). Fatty acids contained in such fat components are mainly linoleic acid, and α-linolenic acid is contained therein in a proportion as low as approximately 7% at highest.

Linoleic acid is converted in the human body to arachidonic acid which is known to cause thrombin in the lungs when administered to rabbits. On the other hand, α-linolenic acid is converted in the human body to eicosapentaenoic acid which is known to possess anti-thrombotic activity. Therefore, the prior-art transfusion emulsions which contain linoleic acid in a larger proportion and α-linolenic acid in a smaller proportion can not be a nutritionally balanced transfusion emulsion. They are unsuitable especially for use in patients who are liable to thrombosis.

As a result of extensive studies, we have found that a transfusion emulsion suitable as an intravenous nutritional supplement is obtained by incorporating α-linolenic acid in a proportion of 10—50 w/v% of the fat component and that such transfusion emulsion possesses a cholesterol-lowering activity as well as an anti-thrombotic activity. The present invention is one completed on the basis of the above findings.

It is an object of the present invention to provide a transfusion emulsion in which fat components are nutritionally balanced.

Another object of the invention is to provide a transfusion emulsion which possesses a cholesterol-lowering activity.

The present invention relates to a transfusion emulsion which is composed of fat, emulsifier and water and in which 10—50 w/v% of the fat component is an α-linolenic acid ester.

As the α-linolenic ester, triglyceride or ethyl ester of α-linolenic acid is preferable. As the oil containing α-linolenic acid in a higher proportion is mentioned, for example, linseed oil. In the present invention, triglyceride of α-linolenic acid synthesized from a α-linolenic acid and glycerin may be employed or linseed oil may be employed after isolating triglyceride of α-linolenic acid or as it is without isolation. Ethyl ester of α-linolenic acid are obtained by reacting α-linolenic acid with ethyl alcohol or by transesterification by reacting triglyceride of α-linolenic acid with ethyl alcohol. As the fat component other than the α-linolenic ester may be used those purified vegetable oils which have been used as the fat component of transfusion emulsions such as purified soybean oil and purified safflower oil.

Any of the emulsifiers which have been used as the emulsifier in transfusion emulsions may be employed in the invention.

In addition, an appropriate amount of an emulsion stabilizer or an emulsion promotor such as glycerin or oleic acid may be incorporated.

The transfusion emulsion of the invention is preferably composed of 5—20 w/v% of the fat component, 1—2 w/v% of the emulsifier and the balance of water, and 10—50 w/v%, preferably 10—30 w/v% of the fat is an α-linolenic ester. The emulsion stabilizer or the emulsion promotor is incorporated usually in the range from 1 to 5 w/v%.

The transfusion emulsion of the present invention is prepared according to conventional procedures. Predetermined amounts of the components are blended, and to the blend is added an alkali. The resulting mixture is dispersed in a homomixer to a homogeneous dispersion, to which distilled water is added. The resulting mixture is emulsified in a high pressure jet emulsifier to give a transfusion emulsion. The transfusion emulsion is divided into plastic bags, which are steam sterilized under high pressure and vacuum packed with film to give the final product.

It was confirmed that the transfusion emulsion of the invention which contained an α-linolenic ester in a higher portion exerted a platelet aggregation-inhibiting action when intravenously administered. Accordingly, the transfusion emulsion of the invention is useful not only as a balanced liquid preparation for nutritional supplement but also as an antithrombotic preparation. Although it is known that α-linolenic esters per se possess a platelet aggregation-inhibitory activity, no antithrombotic transfusion emulsion containing the same in a higher proportion has been disclosed.

Furthermore, the transfusion emulsion of the invention produces an action lowering blood cholesterol level. The cholesterol-lowering action of α-linolenic esters is a novel finding unknown in the literature. The transfusion emulsion is useful for treatment or prevention of arteriosclerosis caused by the cholesterol existing in excess in blood due to excess intake of cholesterol or disturbances of cholesterol metabolism, namely, aortic or coronary sclerosis, myocardial infarct, cerebral thrombosis, aortic aneurysm, obstruction of peripheral arteries, hyperlipemia and diabetes.

For use of the transfusion emulsion of the invention as nutritional supplement, it is administered in exactly the same way as with prior-art transfusion emulsions. For use as an antithrombotic agent or an cholesterol-lowering agent, the transfusion emulsion is administered usually at a dose of α-linolenic ester of 1—30 g, preferably 3—10 g, 200—500 ml in terms of the liquid volume per day in adults, if required, in 1—3 divided doses, the dosage being variable depending upon age, bodyweight and conditions of the patient.

The present invention will be described in more details below with reference to Examples and Test Examples.

## Example 1

A mixture of 50 g of ethyl α-linolenate, 450 g of purified soybean oil, 60 g of purified yolk lecithine and 50 ml of 0.1N-sodium hydroxide is dispersed in a homomixer, followed by addition of injectable distilled water to a total liquid volume of 5 l. The resulting mixture is emulsified in a high pressure jet emulsifier to prepare a transfusion emulsion. The transfusion emulsion is divided into plastic bags in 200-ml portions. The bags are steam sterilized under high pressure at 118°C for 22 min to prepare a transfusion emulsion preparation. After sterilized, the bags are vacuum packed in OV-film (oriented vinylon-film, manufactured by Unitika).

## Example 2

A mixture of 100 g of α-linolenic acid triglyceride, 300 g of purified soybean oil, 48 g of purified yolk lecithine, 2.0 g of oleic acid, 100 g of concentrated glycerin and 10 ml of 0.1N-sodium hydroxide is dispersed in a homomixer, followed by addition of injectable distilled water to a total liquid volume of 4 l. Then, the same treatments as in Example 1 are applied to produce a transfusion emulsion preparation.

## Example 3

A mixture of 80 g of ethyl α-linolenate, 320 g of purified safflower oil, 48 g of purified yolk lecithine, 2.0 g of oleic acid, 100 g of concentrated glycerin and 40 ml of 0.1N-sodium hydroxide is dispersed in a homomixer, followed by addition of injectable distilled water to a total liquid volume of 4 l. Then, the same treatment as in Example 1 are applied to produce a transfusion emulsion preparation.

## Test Example 1

Platelet aggregation-inhibitory activity

Twenty-five Sprague Dawley rats weighing about 200 g were used. They were divided into five 5-rat groups. Rats of Group 1 were intravenously administered at the tail with 18 ml of a commercial transfusion emulsion containing 10 w/v% of soybean oil daily for 8 days. In addition, the rats were freely fed with a solid powder feed (CE-2; manufactured by Nihon Clea Co. Ltd.). Rats of groups 2—4 were daily administered with 18 ml of the transfusion emulsion prepared by the method described in Examples 1 to 3 in the same way as above for 8 days. The solid powder feed was also given ad lib. Rats of Group 5 were freely fed with the solid powder feed. All the animals were fasted for 12 hours after the final intravenous administration and, under 5% Nembutal anesthesia, 4.5 ml was taken from the abdominal aorta by means of a 20 G needle into a syringe in which 0.5 ml of heparin had been placed. A PRP (Platelet Rich Plasma) of 300,000 platelets/μl was prepared for each rat in a conventional manner. In a cuvette was placed 225 μl of the PRP from each rat. To the cuvette, after warmed at 37°C for 5 min, was added 25 μl of collagen, an aggregation inducer to a final concentration of 50 μg/ml and measurement was made for platelet aggregation by means of an aggregometer. The results are given in Table 1. As shown in Table 1, the average rates of aggregation were 60.5%, 55.7% and 64.7% for the rats of Group 2, 3 and 4, respectively, receiving the transfusion emulsion prepared in Examples 1, 2 and 3, respectively. Those data represented a significant inhibition as compared with the rats of Group 1 receiving the commercial transfusion emulsion containing soybean oil and the rats of Group 5 administered an ordinary powder feed only.

EP 0 145 873 B1

### Table 1.

| Group | Transfusion emulsion | Rat | Percent platelet aggregation | Average percent aggregation |
|---|---|---|---|---|
| 1 | Commercial soybean oil transfusion emulsion | 1 | 75.1 | |
| | | 2 | 84.1 | |
| | | 3 | 78.1 | 76.8 |
| | | 4 | 79.4 | |
| | | 5 | 67.6 | |
| 2 | Example 1 | 6 | 59.1 | |
| | | 7 | 61.3 | |
| | | 8 | 72.0 | 60.5 |
| | | 9 | 57.8 | |
| | | 10 | 52.3 | |
| 3 | Example 2 | 11 | 53.7 | |
| | | 12 | 44.8 | |
| | | 13 | 60.3 | 55.7 |
| | | 14 | 73.5 | |
| | | 15 | 46.4 | |
| 4 | Example 3 | 16 | 71.8 | |
| | | 17 | 59.1 | |
| | | 18 | 53.5 | 64.7 |
| | | 19 | 64.0 | |
| | | 20 | 75.2 | |
| 5 | Solid powder feed | 21 | 86.3 | |
| | | 22 | 83.4 | |
| | | 23 | 97.5 | 88.7 |
| | | 24 | 82.5 | |
| | | 25 | 93.8 | |

Test Example 2

Blood cholesterol-lowering action

Groups of 5 male rats (Sprague Dawley) 5 weeks old were used in the experiment. The rats were intravenously administered at the tail each with 15 ml of the transfusion emulsion prepared in Example 1, 2 or 3 above over 1 hour for 4 days. In addition, from the day on which administration of the transfusion emulsion was initiated, 10 ml of 2 w/v% cholesterol suspension in 0.5 w/v% aqueous solution of metrose (Trade name; manufactured by Sin-etsu Chemical Industries Co., Ltd.) was orally administered twice a day for 4 days. After the 4-day administration, the animals was fasted. On the 5th day, blood was drawn from the abdominal aorta, and serum was separated by centrifugal separation and assayed for serum cholesterol by an enzymatic method (Assay Kit: V-cholestase; manufactured by Nissui Pharmaceutical Co., Ltd.). Results are shown in Table 2.

### Table 2.

| Transfusion emulsion | Serum cholesterol (mg/dℓ) |
|---|---|
| Example 1 | 213.7 ± 30.6 |
| Examplè 2 | 198.6 ± 24.7 |
| Example 3 | 221.3 ± 28.1 |
| Comparative Example | 316.5 ± 57.8 |

As clearly seen from Table 2, the antiarterosclerotic preparation containing an α-linolenic acid ester according to the invention exerts a superior blood cholesterol-lowering action to the preparation of Comparative Example. All the preparations of Examples 1, 2 and 3 are significantly different from that of Comparative Example with 5% significance level by the t test.

Acute toxicity

Tests were made in male Sprague Dawley rats to find that the antiarteriosclerotic preparation according to the invention is very safe being similar to commercial transfusion emulsion containing soybean oil. Results are shown in Table 3.

### Table 3.

| Animal (sex) | Route of adminis- tration | Rate of infusion (mℓ/kg/min) | Lethal dose (mℓ/kg) | | | |
|---|---|---|---|---|---|---|
| | | | Example 1 | Example 2 | Example 3 | Comparative Example |
| Rat (male) | Tail vein | 7.5 | 204 | 196 | 187 | 192 |

### Claims

1. Transfusion emulsion comprising fat, an emulsifier and water, characterized in that 10—50 w/v% of said fat component is an α-linolenic acid ester, the emulsion being suitable for intravenous transfusion.

2. Transfusion emulsion for intravenous transfusion according to Claim 1 wherein said α-linolenic acid ester is triglyceride or ethyl ester of α-linolenic acid.

3. Transfusion emulsion for intravenous transfusion according to Claim 1 or 2 wherein said transfusion emulsion is a cholesterol-lowering agent.

### Patentansprüche

1. Zur intravenösen Transfusion geeignete Transfusionsemulsion mit einem Gehalt an Fett, einem Emulgator und Wasser, dadurch gekennzeichnet, daß 10—50 w/v% der Fettkomponente aus einem α-Linolensäureester bestehen.

2. Transfusionsemulsion zur intravenösen Transfusion nach Anspruch 1, dadurch gekennzeichnet, daß der α-Linolensäureester aus einem Triglycerid oder Ethylester der α-Linolensäure besteht.

3. Transfusionsemulsion zur intravenösen Transfusion nach Anspruch 1 oder 2, dadurch gekennzeichnet, daß sie ein cholesterinsenkendes Mittel ist.

### Revendications

1. Emulsion pour transfusion comprenant une graisse, un élmulsifiant et de l'eau, caractérisée en ce que 10 à 50% p/v de ladite graisse constitutive consistent en un ester d'acide α-linolénique, l'émulsion convenant à la transfusion intraveineuse.

2. Emulsion pour transfusion pour transfusion intraveineuse selon la revendication 1, dans laquelle ledit ester d'acide α-linolénique est le triglycéride ou l'ester éthylique de l'acide α-linolénique.

3. Emulsion pour transfusion pour transfusion intraveineuse selon la revendication 1 ou 2, dans laquelle ladite émulsion pour transfusion est un agent abaissant le cholestérol.